# EUROPEAN PATENT APPLICATION

(11) **EP 4 617 358 A1**
(43) Date of publication of application: **17.09.2025**
(21) Application number: 23887225.3
(22) Date of filing: 13.11.2023
(51) Int. Cl.: C12N 1/14, C12R 1/845, A23L 33/195, A23L 29/25, A23L 29/206, A23L 27/00, A23K 10/30

(54) **MYCELIAL BIOMASS, MYCELIAL BIOMASS PRODUCTION PROCESS, USE OF MYCELIAL BIOMASS IN THE PRODUCTION OF FOOD PRODUCTS, MYCELIAL BIOMASS-BASED FOOD PRODUCTS AND PROCESS FOR PRODUCING SAME**

(30) Priority: 13.11.2022 BR 102022023125
(71) Applicant: Mushbites Alimentos Ltda, Ponta Grossa (BR)
(72) Inventor: INAGAKI OSHIRO, Leandro, CEP: 84010-280 Ponta Grossa (BR); DE FRANCISCO, Antonio Carlos, CEP: 84010-270 Ponta Grossa (BR); BITTENCOURT SYDNEY, Eduardo, CEP: 84031-620 Ponta Grossa (BR); UNGER IBRI, Paulo, CEP: 05704-020 São Paulo (BR); ZAGHETTO DE ALMEIDA, Paula, CEP: 05141-000 São Paulo (BR)
(74) Representative: Pereira Garcia, João Luís
(86) International application number: PCT/BR2023/050385
(87) International publication number: WO 2024/098132

(57) **Abstract**

The present invention refers to a mycelial biomass and its production process; The present invention also refers to a food product based on mycelial biomass with high nutritional property and low concentration of additional ingredients and its production process.

## Description

### FIELD OF INVENTION

**.** The present invention refers to a mycelial biomass.

**.** The present invention also refers to a food product based on mycelial biomass with high nutritional properties and low concentration of additional ingredients.

**.** Also, the present invention refers to the process of producing mycelial biomass and the process of obtaining the food product.

### FOUNDATIONS OF INVENTION

**.** In recent years, concern for the environment, sustainability and the search for a healthier and more natural diet, including the reduction or elimination of animal protein consumption, has become increasingly greater.

**.** Studies have projected global population growth to 9.8 billion by 2050, which requires an expansion of the scope, diversity, sustainability and economics of food production.

**.** Currently, the adhesion to veganism or vegetarianism has gained more and more strength, which has resulted in a large increase in the vegetarian and vegan population, and with it the increase in the need for product alternatives that replace animal protein, that is, products from the Plantae and Fungi kingdoms.

**.** Over the past 2 years, the domestic market for alternative protein-based products ("plant-based" foods) has increased dramatically, especially in the category of plant-based chilled meats.

**.** The vast majority of plant-based foods are alternatives to ground meat products of any animal species, such as sausages, hamburgers, and nuggets. Data compiled by the U.S. Agricultural Marketing Service (AMS) shows that only 36% of animal protein meat products are minced meat, while the rest of the products consist of whole products such as steak/steak.

**.** Since the 1960s, textured soy protein has served as a substitute for minced/ground meat products. The same technology used to obtain textured soy protein was used to obtain other textured vegetable proteins, such as wheat and peas.

**.** Fungi-derived protein sources, also known as mycoproteins, are affordable alternatives that have gained attention from large and small businesses.

**.** Although mycoprotein contains less protein than animal meat, mycoprotein has higher amounts than those derived from plants and its composition has a large amount of fiber, providing, on average, about 6 grams of fiber for every 100 grams of food. It is worth mentioning that protein from fungi is a great nutritional quality; it has a low amount of sodium, sugar and fat; it is rich in essential amino acids, vitamin B12, B9, calcium, phosphorus, magnesium and zinc. In addition to having lower carbon emissions and lower water consumption, when compared to the production of beef and chicken.

**.** The kingdom Fungi has an estimated diversity of 1.5 million species, 117 of which are traditionally used as food.

**.** The production of fungal/mycelial biomass for use in the food supply can be divided into two categories: (a) mushroom production, which has been practiced for thousands of years, which requires cultivation cycles long enough to produce the fruiting body and is limited in shape and final sizes; and (b) the production of mycoproteins by (I) liquid culture (introduced in the 1980s as QuornTM), which results in a fungal cell paste without any fiber alignment and thus requires processing to create a cohesive and acceptable texture or (II) solid-state culture, which facilitates the achievement of edible fungal/mycelial biomass with cohesive structure and texture. Both ways of obtaining mycoproteins can offer unique nutrient profiles, as well as sensory and texture suitable for meat alternatives.

**.** Additionally, unlike large animal protein (animal meat) industries, the production of fungal protein (mycoprotein) requires little space and allows a variety of textures to be obtained from different types of meats, which is not possible to obtain with plant-based proteins (soybean, wheat and pea).

**.** Therefore, faced with this promising scenario, large, medium and small companies have begun to see the use of fungi for the production of alternative food to meat as an alternative, which in addition to being characterized as a vegan option, contributes to reducing deforestation caused by cattle ranching by half.

**.** To obtain a meat substitute product, composed of fungal mycelium, it is necessary to grow the fungus in a process usually called fermentation. This fermentation is a bit similar to the one used to create beer, and uses sugars and other nutrients in the process. In addition, the filamentous nature of the fungus causes the process to create fibrous bundles, called hyphae, which if processed in a specific way resemble the texture of animal flesh. The fungi most commonly used for the production of protein made from fungi are *Fusarium venenatum, Fusarium flavolapis, Neurospora intermedia, Neurospora glabra.*

**.** Faced with this scenario, several researchers have been working to improve the process of cultivating fungi, to obtain mycelia with desirable structure and texture, to improve processes for obtaining food based on fungal mycelia, for scaling up and reducing costs, as well as studies with other species of filamentous fungi to seek new alternatives for use in the production of mycelia for use in the food industry.

**.** As can be seen in the state of the art, the PI7800368 document describes a process for texturing a fungal mass of mycelium with defined steps and subsequent freezing.

**.** The document WO2021092051 describes an improved mycelium suitable for use as a food product. While the document US20200305486 describes a food comprising particles of a filamentous fungus of the genus *Fusarium,* agar and an edible hydrocolloid, and the document WO2020061502 describes method of production of protein foods based on filamentous fungal mycelium.

**.** The teachings of US20190373934 reveal systems to cultivate fungal mycelium and an edible meat substitute product containing fibrous mycelium mass in defined weight %.

**.** Thus, focusing on this new need and seeking improvements in relation to the state of the art, including the development of edible product alternatives that can replace animal protein, the researchers of this invention developed a mycelial biomass for the preparation of a food product with high nutritional properties. In addition to developing a simple process, easy to scale and low production cost.

**.** It is important to emphasize that, in the state of the art, there is no solution equivalent to the one presented by this invention, which combines technical differentials, obtaining a *"clean label"* product, simpler production process, economic advantages and preservation of the environment.

### SUMMARY OF THE INVENTION

**.** The main objective of this invention is to provide a mycelial biomass with high nutritional properties and its production process.

**.** In a second aspect, the present invention aims to use mycelial biomass as a basis for the production of a food product. Mycelial biomass, the object of this invention, allows the obtaining of a food product, a substitute for animal meat, with a high protein index, low caloric value and low amount of fat, in which 95% or more of the product corresponds to mycelial biomass.

**.** The food product based on mycelial biomass and its production process is still the object of this invention.

### BRIEF DESCRIPTION OF THE FIGURES

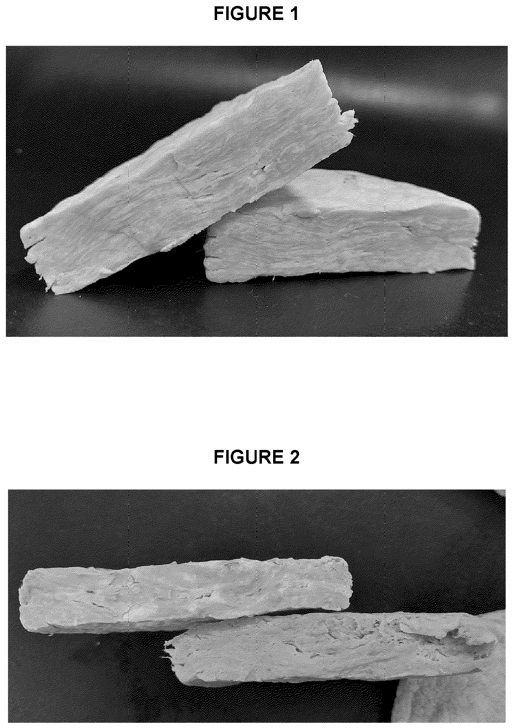

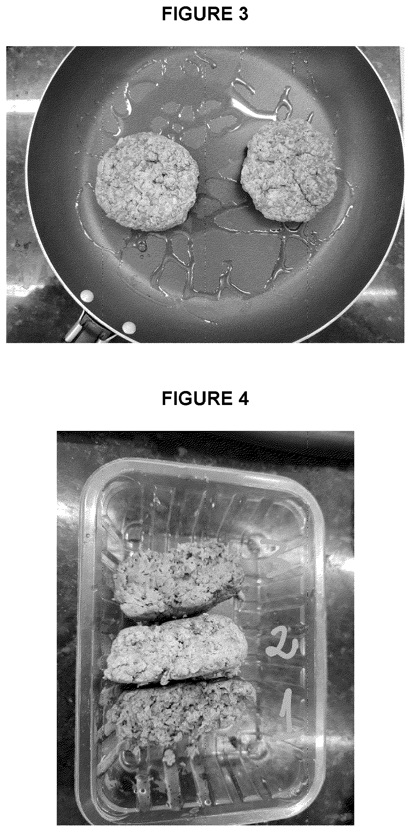

**.** Figure 1 illustrates the food product containing the mycelial biomass obtained by the cultivation process using culture medium 1.
**.** Figure 2 illustrates the food product containing the mycelial biomass obtained by the culture process using culture medium 3.
**.** Figure 3 illustrates a hybrid burger 50:50 beef and mycelial biomass obtained by the process of this invention (left) and 100% beef (right).
**.** Figure 4 illustrates a visual comparison between products: 3 - 100% beef burger, 2 - product of figure 3 left without addition of natural coloring, 1 - product of figure 3 left with addition of 1% natural beet coloring

### DETAILED DESCRIPTION OF THE INVENTION

**.** The main objective of this invention is the realization of a mycelial biomass with high nutritional properties and its use as a basis for the production of a food product. Mycelial biomass, the object of this invention, allows its pressing in a single step, resulting in the obtaining of cuts that simulate chicken breast, without the need for cuts of the pressed material and subsequent new pressings, as described in the state of the art.

**.** The present invention also aims at the process, the process of obtaining mycelial biomass and the process of preparing the food product.

**.** In addition, mycelial biomass, the object of this invention, is the basis for the production of a food product, a substitute for animal meat, with a high protein index, low caloric value and fat index.

**.** Finally, this invention aims to materialize the food product based on mycelial biomass and its production process.

**.** Biomass, the object of this invention, is composed of a mass of filamentous fungus without the presence of chemical additives. The biomass is composed of 100% filamentous fungus mass and does not contain the addition of chemical additives.

**.** The absence of chemical additives in mycelial biomass results in a biomass classified as *"clean-label"* and will allow the food product to be obtained as *"clean label".*

**.** According to this invention, *"clean label"* products are those produced with few ingredients and that do not have chemical additives such as dyes, preservatives, stabilizers.

**.** According to this invention, the filamentous fungi used to obtain biomass are: *Pleurotus ostreatus, Ganoderma lucidum, Rhizopus oligosporus, Rhizopus microsporus var. oligosporus, Rhizopus oryzae* being preferentially the fungus *Rhizopus oligosporus.*

**.** And the biomass production process, also the object of this invention, is easy, has a fast execution, is easily scaled, in addition to having a low production cost.

**.** Mycelial biomass is obtained from the process containing the stages of filamentous fungus cultivation, filtration, rinsing, inactivation of the fungus.

**.** For the production of biomass, object of this invention, filamentous fungi selected, but not limited, from the group consisting of: *Pleurotus ostreatus, Ganoderma lucidum, Rhizopus oligosporus, Rhizopus microsporus* var*. oligosporus, Rhizopus oryzae* being preferentially the fungus *Rhizopus oligosporus.*

**.** To obtain fungal mycelial biomass, it is necessary to cultivate the fungus through a specific process to allow the filamentous nature of the fungus to create fibrous bundles that allow the formation of a biomass with characteristics that resemble the texture of animal flesh

**.** To obtain mycelial biomass, the following steps are necessary:
(a) Growth of the fungus in solid medium;
(b) Pre-inoculum;
(c) Fermentation in a bioreactor;
(d) Filtration of filamentous fungus mass;
(e) Biomass washing/rinsing; and
(f) Inactivation of the fungus

**.** In the growth stage in solid medium, the fungus is cultivated in BDA medium "Potato Dextrose Agar" in inclined agar tubes for a period of 4 to 10 days at a temperature of 25 to 39°C in an incubator, preferably from 30 °C to 35°C. The fungus used is a filamentous fungus selected from the group consisting of: *Pleurotus ostreatus, Ganoderma lucidum, Rhizopus oligosporus, Rhizopus microsporus var. oligosporus, Rhizopus oryzae, Neurospora intermedia, Neurospora glabra, and preferably the fungus Rhizopous oligosporus.*

**.** After growth in solid medium, in the pre-inoculum stage the spores are suspended in sterile distilled water and inoculated at a concentration of 105 spores per mL of liquid culture medium. For this step, 500 mL Erlenmeyer flasks are used. The culture is kept at a temperature of 30 to 38°C, preferably at 35°C, under agitation of 130 rpm for 15 to 24 hours in an incubator with orbital agitation.

**.** In this step, defined liquid culture media are used, which may comprise (i) carbon sources: mono, di, tri and polysaccharides, preferably corn starch, (ii) nitrogen sources: yeast extract, malt extract, hydrolyzed or non-hydrolyzed grain bran, ammonia and its salts, preferably brewery residual yeast, (iii) micronutrients as sources of magnesium, phosphorus and potassium, preferably magnesium sulfate, monobasic potassium phosphate and/or their mixtures and are responsible for the growth of the fungus with the desired characteristics to obtain the mycelial biomass, object of this invention. The liquid culture media of primary interest are composed of the following mixtures:
- Culture Medium 1 comprises corn starch, ammonium nitrate, magnesium sulfate, monobasic potassium phosphate and yeast extract.
- Culture Medium 2 comprises corn starch, malt extract, yeast extract, and magnesium sulfate.
- Culture Medium 3 comprises corn starch, malt extract, yeast extract, and magnesium sulfate.
- Culture medium 4 is comprises brewery waste yeast and corn starch

**.** Preferably, liquid culture media are:
- Culture Medium 1 comprises 20 to 60 g/L of corn starch, 0.125 g/L of ammonium nitrate, 0.2 g/L magnesium sulfate, 0.05 g/L monobasic potassium phosphate, and 5 to 20 g/L of yeast extract. Preferably 50 g/L of corn starch and 12g/L of yeast extract.
- Culture Medium 2 comprises 20 to 60 g/L of corn starch, 2 to 25 g/L of malt extract, 5 to 20 g/L of yeast extract, and 0.2 g/L magnesium sulfate. Preferably 50 g/L of corn starch, 2 g/L of malt extract and 12 g/L of yeast extract.
- Culture Medium 3 comprises 20 to 60 g/L of corn starch, 2 to 25 g/L of malt extract, 5 to 20 g/L of yeast extract, and 0.2 g/L magnesium sulfate. Preferably, 50g/L of corn starch, 25 g/L of malt extract, 4 g/L of yeast extract.
- Culture medium 4 comprises 50% (v/v) of residual brewery yeast and 20 g/L of corn starch.

**.** The process using Culture Medium 1 or 2 yields about 12 grams of dry mycelium per liter; The use of culture medium 3 yields about 11 grams of dry mycelium per liter and the use of culture medium 4 yields about 14 grams of dry mycelium per liter.

**.** After this period, the culture is transferred to a bioreactor for the fermentation stage. To add to the reactor, a pre-inoculum suspension is prepared in the ratio of 10% of the pre-inoculum to 90% of sterile fresh culture medium. The suspension is transferred to the bioreactor and the aeration is adjusted. To obtain the desired biomass, the aeration is adjusted from 0.5 to 3 VVM, the bioreactor using contains a set of blades of the flat blade type near the air inlet and the elephant ear type of downflow at the top, optimizing the oxygen transfer and the mixing time of the system. The pre-inoculum suspension is maintained in the bioreactor for a period of 24 to 48 hours without the need for pH control throughout the process. The absence of the need for pH control helps to make the process easier and cheaper.

**.** On the other hand, if it is of interest, pH control can be carried out.

**.** In the filtration step, the biomass generated in the above step is separated from the fermented medium by simple filtration using filter material with porosity of 5 to 17 mesh. A filter material with a porosity of 14 mesh is preferred.

**.** The biomass formed is rinsed/washed under running water until the pH is neutralized. This step aims to remove all residues from the culture media used in the process.

**.** Additionally, aiming at the inactivation of the fungus, reduction of the RNA content and extension of the shelf life of the product, the mycelial biomass goes through a heating stage, at 70°C for a period of 2 to 45 minutes, preferably from 10 to 15 minutes, preferably 15 minutes, and then the mycelial biomass is ready for pressing, freezing and/or use in the production of the food product

**.** The fungal mycelial biomass formed is rich in fiber, has a high protein content and very low energy value and fat index when compared to similar products of plant origin (known as *"plant-based"*).

**.** According to this invention, the mycelial biomass obtained at the end of the pressing process has at least 1 % to 10 %, i.e. 1 g to 10 g of fibre for every 100 g of portion. While animal protein does not have any fiber in its composition.

**.** The fungal mycelial biomass has 60 to 150 kcal, with a preferential 78 kcal and less than 4g of fat per 80 g portion of biomass. This represents very low energy value and very low fat content when compared to similar *plant-based* products or traditional chicken meat. A similar *plant-based* product on the market has around 150 Kcal, practically double the 78 kcal of biomass, and a traditional chicken breast has around 126kcal.

**.** Since the *market-leading plant-based* product has 10.8 g of fat for each 80 g portion of product and a traditional chicken has 3.5 g of fat for 80 g of product, that is, the *plant-based* product has 10 times more fat than fungal mycelial biomass and traditional chicken has 3.5 times more fat when compared to fungal mycelial biomass, object of the present invention.

**.** Regarding the protein content, the biomass, object of this invention, has a high protein content when compared to the calories that the product delivers to the consumer. In the same example above, biomass with a moisture content of 78% has, on average, 10.5 g of protein for every 78 Kcal, that is, a ratio of 13.5% of protein to calories. As the *market-leading plant-based* product , it has 11 g of protein for every 150 Kcal, that is, a ratio of 7% protein to calories.

**.** Mycelial biomass, the basis of the food product, is composed, on average, of: Aspartic Acid 0.70%; Glutamic Acid 0.86%; Serine 0.30%; Glycine 0.40 %; Histidine 0.22%; Taurine <0.01 (LQ); Arginine 0.42%; Threonine 0.33%; Alanine 0.46%; Proline 0.24%; Tyrosine 0.29%; Valine 0.44 %; Methionine 0.10%; Cystine 0.07%; Isoleucine 0.38%; Leucine 0.53%; Phenylalanine 0.32%; Lysine 0.74%; Hydroxyproline <0.01 (LQ)%; Sum of Total Amino Acids 6.81 % and Carbohydrates 7.00 g/100g. The Energy value is 78.12 KCal/100g; Humidity and Volatiles 78.11%; Crude Protein 10.46%; Ether Extract 0.92%; Dietary Fiber 2.09 g/100g; Insoluble Dietary Fiber 2.09 g/100 g; Soluble Dietary Fiber <0.10 g/100 g; Mineral Matter 1.42 %. This gives biomass high nutritional properties.

**.** In addition, the following mycotoxins were not detected: Total Aflatoxins (B1+B2+G1+G2) and Ochratoxin; and heavy metals: Arsenic; Cadmium; Lead and Mercury.

**.** Mycelial biomass is the basis for the production of a food product with high nutritional properties and which has a visual appearance and texture similar to that of chicken meat. The product can be flavored.

**.** To obtain the food product based on mycelial biomass, the following steps are necessary:
(a) Preparation of mycelial biomass
(b) Pressing
(c) Addition of the additional components
(d) Heating and
(e) Freezing.

**.** In the mycelial biomass preparation step, the biomass is obtained as described above.

**.** After preparation, the biomass obtained is placed in molds in the desired shape (for example, chicken breast) with perforations and is pressed from 10 to 100 psi, preferably 20 psi.

**.** In the addition step of the additional components, the pressed biomass is submerged in a solution containing the flavoring, chickpea flour and gum arabic until the product absorbs 10% of its weight from the solution. The final concentrations of the product can contain from 0 to 4% acacia gum, from 0.5 to 4% chickpea flour, from 0.05 to 0.7% of flavoring.

**.** Finally, the product obtained is heated to 70°C for 15 minutes and minutes and frozen in a common freezer at -12°C and -15°C. This step can be performed as a step before freezing or before pressing.

**.** The food product obtained simulates an entire cut of chicken breast, has a neutral color similar to the color of chicken breast, has more than 50% of the hyphae fibers aligned horizontally, as illustrated in figures 1 and 2. The product has a texture of 35 to 40 N WBSF (Warner-Bratzler Shear Force).

**.** In addition, the food product, object of this invention, is composed of 90 to 99% mycelial biomass in relation to the total weight of the product and 1 to 10% of additional compounds in relation to the total weight of the product. More preferably, the food product is composed of 95% or more mycelial biomass in relation to the total weight of the product, preferably of the fungus *Rhizopus oligosporus,* and 5% or less of other components in relation to the total weight of the product.

**.** According to the present invention, additional compound is understood to be acacia gum, starch, chickpea flour, flavoring, bamboo fiber, oat fiber, xanthan gum, coconut oil, corn oil or any other vegetable oil of neutral flavor, olive oil and/or their mixtures. Such a mixture of compounds is present in the proportion of 5% or less of the total weight of the product. The additional component of preference is a mixture of acacia gum, chickpea flour, flavoring, and/or their mixtures. The mixture comprises 0 to 4 % acacia gum, 0.5 to 4 % chickpea flour, 0.05 to 0.7 % flavouring. Preferably, the mixture comprises acacia agoma at 2%, chickpea flour at 1.5% and flavouring at 0.2%. The mixture used can be in the form of a solution or suspension.

**.** The food product obtained has a moisture content of 65 to 90 % and can be directly frozen and stored in a common freezer at -12 to -15°C.

**.** Having described an example of a preferred realization of the present invention, it must be understood that the scope of the present invention encompasses other possible variations of the inventive concept described, being limited only by the content of the claims only, including the possible equivalents.

### EXAMPLE 1: Fungus cultivation process

**.** The fungus *Rhizopus oligosporus* is inoculated in solid BDA medium "Potato Dextose Agar" in inclined agar tubes and incubated for 7 days at 30°C in an incubator. Then the grown spores are resuspended in sterile distilled water. An amount of approximately 105 spores per mL are transferred to 200 mL of liquid medium in Erlenmeyer for the composition of the preinoculum and maintained at a temperature of 35°C. The liquid medium used is composed of liquefied corn starch (50 g/L); malt extract (2 g/L); yeast extract (12 g/L); MgSO4.7h2O (0.2 g/L). The pre-inoculum is incubated under 130 rpm shaking at 35°C for 24 hours in an orbital incubator. The culture is transferred to a bioreactor for the fermentation stage in the amount of 10% pre-inoculum and 90% fresh culture medium. To obtain the desired biomass, aeration is set to 0.75 VVM until the dissolved oxygen in the medium reaches 50 % saturation, and then it is set to 1.5 MVV. using a set of flat blades near the air inlet and elephant ear type blades. The culture is kept in the bioreactor for 44 hours, the pH is kept at 5.5.

### EXAMPLE 2: Process of obtaining biomass

**.** The mycelial biomass obtained in example 1 is filtered in a filter with a 14 mesh. The biomass is rinsed under running water to dispose of the residues from the growing medium.

**.** Mycelial biomass is heated to 70°C for 10 minutes and can be pressed and frozen or used in the production of the food product described in example 3.

### EXAMPLE 3: Process of obtaining the food product

**.** The mycelial biomass obtained in example 2 is submerged in a solution containing acacia gum (2%), chickpea flour (1.5%) and flavoring (0.2%). The biomass is in contact with the solution for 15 minutes.

**.** The mycelial mass is then transferred to a stainless steel mold in the shape of a chicken breast with perforations on the bottom and sides. The dough is then pressed until the final moisture of the product is at 80%. The final product will have aligned fibers and texture between 35-40 N (WBSF). The sample is then heated to 70°C for at least 10 minutes and then frozen in a common freezer.

### EXAMPLE 4: Sensory testing

**.** Two blinded sensory tests were performed with 7 participants each. Test 1 aims to evaluate the addition of components to improve the texture of the product. Test 2 aims to evaluate the mixture of components to improve the texture of the product. For each test the participants tasted the products and were asked to give a score from 1 to 10 on how pleasant the products were for the: chewiness, firmness, texture and taste. The means are presented in tables 1 and 2.

**.** In experiment 1 (results in table 1), biomass plus low concentration (1%) and high concentration (4%) of bamboo fiber 90, bamboo fiber 200 and acacia gum were tested. The experiments with 90 bamboo fiber and 200 bamboo fiber resulted in worse evaluations, since the product became more brittle and with a dry appearance. The acacia gum experiments led to an overall improvement in the scores assigned by the participants.

**TABLE 1 - Sensory test for the evaluation of additives to change the texture of the product**

| Sample* | Grade Points Averages from 1 to 10 | | | |
|---|---|---|---|---|
| | Chewiness | Firmness | Texture | Taste |
| Control** | 6 | 7 | 5,7 | 5,3 |
| Acacia Gum 1% | 6,8 | 7,7 | 6,2 | 7,1 |
| Acacia Gum 4% | 6,6 | 8,2 | 6,8 | 6,3 |
| Bamboo Fiber 90 1% | 5,4 | 6,1 | 4,2 | 5,1 |
| Bamboo Fiber 90 4% | 3,2 | 6 | 3,8 | 5,6 |
| Bamboo Fiber 200 1% | 4,4 | 6,1 | 4,1 | 5,6 |
| Bamboo Fiber 200 4% | 3,6 | 5,2 | 3,4 | 5 |

**.** In experiment 2 (results in table 2), 3 mixtures of acacia gum, bamboo fiber, chickpea flour and flavoring were used in the following concentrations:
- Blend A: Acacia Gum 2%, Bamboo Fibre 0.2%, Chickpea Flour 1%, Natural or Identical to Natural Flavouring Powder Chicken, Garlic & Onion Flavour, 0.2%.
- Blend B: Acacia gum 2%, Bamboo fibre 0, Chickpea flour 1%, natural or identical to natural flavouring in chicken, garlic and onion flavour powder, 0.2%.
- Blend C: Acacia gum 2%, Bamboo fibre 0.2%, Chickpea flour 0, natural flavouring or identical to natural chicken flavour powder, garlic and onion, 0.2%.

**.** The concentration of 2 % of acacia gum was maintained, with or without the addition of chickpea flour (1%) and 90 bamboo fiber (0.2%). It can be observed that the addition of chickpea flour resulted in better acceptance of the product, while bamboo fiber, even at low concentration, resulted in worse evaluations. Based on this study, chickpea flour and acacia gum are used in the food product.

**TABLE 2 - Sensory test for the evaluation of the mixture of additives to improve texture**

| Sample | Grade Points Averages from 1 to 10 | | | |
|---|---|---|---|---|
| | Chewiness | Firmness | Texture | Taste |
| The | 8,6 | 9,2 | 8,8 | 8,1 |
| B | 9,3 | 9,6 | 9,4 | 8,4 |
| C | 8 | 8,4 | 7,7 | 7,4 |

### EXAMPLE 5: Pilot-scale mycelium production

**.** In a bioreactor with 200L of useful capacity equipped with a stirring system composed of a Rushton type paddle and an elephant ear type blade, it was inoculated with 10% (v/v) pre-inoculum as described in EXAMPLE 1.

**.** Cultivation in the bioreactor was conducted for 38 hours with pH control at 5.5 and constant aeration rate. Different rates of aeration and mechanical agitation were evaluated. The process yield was compared with the maximum yield obtained at laboratory scale and expressed as % (50% yield means that that condition produced half of the maximum possible biomass according to the tests performed at laboratory scale).

**TABLE 3 - Comparison between process yield and maximum yield obtained**

| **Experiment** | **Agitation (RPM)** | **Aeration (vvm)** | **Yield** |
|---|---|---|---|
| 1 | 25 | 0,2 | 50% |
| 2 | 22 | 0,25 | 53% |
| 3 | 44 | 0,375 | 43% |
| 4 | 22 | 0,5 | 74% |
| 5 | 22 | 0,75 | 83% |
| 7 | 30 | 0,75 | 95% |
| 8 | 22 | 0,75 | 90% |
| 9 | 22 | 0,75 | 96% |
| 10 | 40 | 0,75 | 69% |
| 11 | 22 | 1 | 75% |
| 12 | 22 | 2 | 68% |

**.** On a pilot scale, it was noted that agitation above 40rpm resulted in reduced biomass production. This must have occurred due to the mechanical stress suffered by the microorganism. The increase in air injection into the system also had a similar effect, especially above 0.75vvm.

### EXAMPLE 6: Inoculum Rate Study

**.** Different inoculum rates were evaluated to start fermentation in a pilot bioreactor with a useful volume of 200L. Pre-inoculum cultures were prepared as explained in example 1. The experiments and pilot bioreactor were carried out in triplicate and under the same rates of aeration, pH and mechanical agitation.

**.** Preinoculum can be made from a spore solution containing 10^5 to 10^10 spores/mL, preferably 10^8, or from a liquid culture at a ratio of 2-10%, preferably 6%.

**.** The biomass production on a dry basis was quantified at the end of fermentation. With an inoculum rate of 10%, 12g/L of mycelium were produced; while 11.46g/L was obtained using 6% inoculum rate and 8.33g/L with 2%. The possibility of using low inoculum rates favors the scaling of the technology. In the case of an inoculum rate of 10%, for example, a 200L culture could inoculate 2,000L of medium, while an inoculum rate of 6% would allow inoculating 3,333L.

**.** In a second batch of experiments, a bioreactor containing 200L of culture medium was inoculated with a spore solution containing 10^5, 10^8 and 10^10 spores. Under these conditions, the fermentation process lasted from 50 to 55 hours until the total consumption of sugars from the culture medium, differing in the amount of mycelium biomass produced. Compared to the condition of higher initial spore concentration, the cultivation that started with 10^8 spores resulting in the formation of 95% of biomass and the one that started with 10^5 resulted in 80%.

### EXAMPLE 7: Production of protein concentrate from dehydrated mycelium

**.** The fresh biomass obtained can be processed to remove moisture, resulting in a dry protein concentrate. Dewatering can be performed by different methods such as drying in trays, fluidized bed or spouted bed, spray dryer, freeze-drying, natural (solar), rotary dryers, tunnels, conveyors, cyclones, screw advances, among others. In this process, the process temperature should preferably be low (preferably between 50 and 60°C) when it is desired to preserve the nutritional and sensory properties of the mycelium, avoiding protein hydrolysis, thermal destruction of vitamins and lipid oxidation. Can be used at room temperature up to 100°C.

**.** In addition, the biomass obtained can be later processed in a mill for standardization of granulometry.

**.** In this stage, 5kg of fresh mycelium biomass was subjected to drying in trays, in an oven with forced air circulation, at 50°C for 24 hours. At the end, the dry material was processed in a disc mill and analyzed for nutritional composition.

**TABLE 4 - biomass nutritional data**

| **Essay** | **Result** |
|---|---|
| Energy value | 290,09 |
| Carbohydrates | 31,73 |
| Protein | 36,00 |
| Total Fat | 2,13 |
| Saturated Fat | 0,76 |
| Trans gurdura | <LQ <0.05 |
| Dietary Fiber | 9,84 |
| Sodium | 53,69 |
| Moisture | 13,52 |
| Ashes | 6,78 |

**.** While drying adds the advantage of increasing the shelf life of the product and avoids dependence on the cold/frozen chain for storage and transportation of the product, it profoundly alters the texture of the product which, when rehydrated, does not return to the original fibrous texture.

### EXAMPLE 8: Application in meat products

**.** A hybrid product was prepared by mixing red meat with fresh mycelium biomass with 80% moisture content illustrated in figures 3 and 4 in comparison with a product comprising only meat and also in the presence and absence of dye. Different proportions of beef and mycelium were evaluated. As a standard (comparative) product, a hamburger was prepared using 100% beef. In the first test, 100g hamburgers were produced manually, to which 1% of natural beet dye was added. A sensory test was then performed with 12 people (Table 5).

**.** It was noted that maintaining a proportion of at least 50% beef results in great acceptability of the product. Below this content, some loss of chewiness and texture is noticeable. The worse perception of flavor can be justified by the fact that no flavoring was used in this formulation. Also, lower scores in relation to appearance are related to color perception, since the beet dye results in a pinker product than the brownish tone of cooked meat, giving the impression of artificiality.

**TABLE 5 - Sensory test**

| Proportion Red meat: mycelium (%) | Grades (0 to 10) | | | |
|---|---|---|---|---|
| | Chewiness | Texture | Flavor | Appearance |
| 100:0 | 10 | 10 | 9,8 | 10 |
| 70:30 | 10 | 9.8 | 9,5 | 10 |
| 60:40 | 9,8 | 9,8 | 9 | 10 |
| 50:50 | 9,9 | 9,5 | 9,2 | 10 |
| 40:60 | 9,4 | 9,2 | 8,5 | 9 |
| 30:70 | 8,5 | 9,2 | 8 | 8 |

### EXAMPLE 9: REDUCING ENVIRONMENTAL IMPACTS

**.** Agriculture and livestock are currently responsible for 70% of water consumption and 25% of greenhouse gases emitted by human activities. Animal production stands out because it depends directly on agricultural production and carries in its life cycle all the environmental impacts caused by it, such as the use of fertile land, chemicals (fertilizers, fertilizers, insecticides, etc.), for example. It also contributes massively to methane emissions, consumption of water resources, energy consumption, pollution of water bodies, production of liquid and solid effluents that are difficult to treat, and soil disposal and degradation. Agriculture and livestock are also closely related to deforestation, because their production is horizontal and the expansion of productive areas requires the advance into new territories.

**.** Cattle, chicken and pork are currently the most consumed sources of animal protein in the world. In the case of cattle, only 40-45% of their weight is meat, of which 25% is protein on average. A cattle is slaughtered after 24 months and occupies approximately 15m² of area when confined. Considering an animal slaughtered with 650kg (average in 2021), it will have produced only 0.006kg of protein/day/m². The water footprint of beef production is estimated at 10.5m³ per kg of meat, or 42m³ per kg of protein.

**.** With the productivity obtained by the process described in this document, we can obtain 12g of dry mycelium biomass per m³ of culture medium. The resulting biomass has 48% protein content on a dry basis and is generated in 24 hours, which represents 5.76kg of protein per day for each m³ of culture medium. Considering a bioreactor with a useful volume of 10m³ with a height/diameter ratio of 3, we can achieve a protein productivity of approximately 58.5kg of protein/day/m², or almost 10,000x higher than the productivity of beef. Regarding the water footprint, we estimate that this technology will use at least 10.5x less water (a calculation that can be even lower if you consider the use of industrial effluents as part of the growing medium).

## Claims

1. MYCELIAL BIOMASS WITH HIGH NUTRITIONAL PROPERTIES **CHARACTERIZED BY** COMPRISING A MASS OF FILAMENTOUS FUNGUS WITHOUT CHEMICAL ADDITIVES, BEING THE FILAMENTOUS FUNGUS SELECTED FROM THE GROUP CONSISTING OF: *Pleurotus ostreatus, Ganoderma lucidum, Rhizopus oligosporus, Rhizopus microsporus var, oligosporus, Rhizopus oryzae.*

2. MYCELIAL BIOMASS, according to claim 1, **CHARACTERIZED BY** the filamentous fungus being *Rhizopus oligospourus.*

3. MICELIAL BIOMASS, according to claims 1 or 2, **CHARACTERIZED BY** containing 1 % to 8 % of fiber, 60 to 90 kcal and less than 1 g of fat per 80 g of biomass and high protein content, containing, on average, 10.5 g of protein for every 78 Kcal.

4. MICELIAL BIOMASS, according to claims 1 to 3, **CHARACTERIZED BY** comprising, on average: Aspartic Acid 0.70%; Glutamic Acid 0.86%; Serine 0.30%; Glycine 0.40 %; Histidine 0.22%; Taurine <0.01 (LQ); Arginine 0.42%; Threonine 0.33%; Alanine 0.46%; Proline 0.24%; Tyrosine 0.29%; Valine 0.44 %; Methionine 0.10%; Cystine 0.07%; Isoleucine 0.38%; Leucine 0.53%; Phenylalanine 0.32%; Lysine 0.74%; Hydroxyproline <0.01 (LQ)%; Sum of Total Amino Acids 6.81 % and Carbohydrates 7 g/100 g; the Energy value being 78.12 KCal/100 g; Humidity and Volatiles 78.11%; Crude Protein 10.46%; Ether Extract 0.92%; Dietary Fiber 2.09 g/100 g; Insoluble Dietary Fiber 2.09 g/100 g; Soluble Dietary Fiber <0.10 g/100 g; Mineral Matter 1.42 %.

5. PRODUCTION PROCESS of biomass as defined in any of the claims 1 to 4 **CHARACTERIZED BY** comprising the stages of:
(a) Growth of the fungus in solid medium;
(b) Pre-inoculum;
(c) Fermentation in a bioreactor;
(d) Filtration of filamentous fungal mass;
(e) Washing/rinsing of the dough;
(f) Inactivation of the fungus; and
(g) drying.

6. PRODUCTION PROCESS, according to claim 5, **CHARACTERIZED BY** in the growth stage in solid medium the fungus is inoculated in BDA medium "Potato Dextose Agar" in agar tubes and kept inclined for 4 to 10 days at a temperature of 25 to 39°C.

7. PRODUCTION PROCESS, according to claim 5, **characterized by** the fungus used is a filamentous fungus selected from the group consisting of *Pleurotus ostreatus, Ganoderma lucidum, Rhizopus oligosporus, Rhizopus microsporus var, oligosporus, Rhizopus oryzae, Neurospora intermedia, Neurospora glabra.*

8. PRODUCTION PROCESS, in accordance with claim 5, **CHARACTERIZED BY** the liquid culture medium comprising corn starch, yeast extract, malt extract, brewery residual yeast, ammonium nitrate, magnesium sulfate, monobasic potassium phosphate and/or their mixtures.

9. PRODUCTION PROCESS, according to claim 5, **CHARACTERIZED BY** the fermentation stage comprises: the preparation of a pre-inoculum suspension in the proportion of 2 to 10% of the pre-inoculum to 90 to 98% of sterile fresh culture medium; transfer of the pre-inoculum suspension to the bioreactor; adjustment of aeration to 0.5 to 3 VVM; maintenance of the pre-inoculum suspension in the bioreactor for a period of 24 to 48 hours without the need for pH control.

10. PRODUCTION PROCESS, according to claim 5, **CHARACTERIZED BY** in the filtration step the biomass generated in the previous step is separated from the fermented medium by simple filtration using filter material with porosity of 5 to 17 mesh.

11. PRODUCTION PROCESS, according to claim 5, **CHARACTERIZED BY** the fact that in the rinsing/washing stage the biomass formed is rinsed under running water until the pH is neutralized and all residues of the culture media used in the process are removed.

12. PRODUCTION PROCESS, according to claim 5, **CHARACTERIZED BY** in the inactivation stage of the fungus, the biomass is heated to 70°C for 10 to 15 minutes.

13. PRODUCTION PROCESS, according to claim 5, **CHARACTERIZED BY** the drying step taking place in trays, fluidized bed or spouting bed, spray dryer, freeze-drying, natural (solar), rotary dryers, tunnels, conveyors, cyclones, screw advancement.

14. PRODUCTION PROCESS, according to claims 1 to 13, **CHARACTERIZED BY** the biomass obtained is pressed and frozen or frozen for subsequent pressing.

15. PRODUCT partial or total substitute of animal protein, **CHARACTERIZED BY** comprising: 40 to 99% of mycelial biomass described in claims 1 to 4 in relation to the total weight of the product, 0 to 50% of meat and 1 to 10% of additional compounds in relation to the total weight of the product.

16. PRODUCT in accordance with claim 15, **CHARACTERIZED BY** the additional compound being selected from the group consisting of acacia gum, chickpea flour, flavoring, bamboo fiber, oat fiber, xanthan gum, coconut oil, corn oil or any other vegetable oil of neutral flavor, and/or their mixtures.

17. USE of the biomass described in claims 1 to 4, **CHARACTERIZED BY** BEING to prepare a food product that is a partial or total substitute for animal protein.

18. PRODUCT PRODUCTION PROCESS as defined in claims 15 or 16, **CHARACTERIZED BY** comprising the stages of:
(a) Preparation of mycelial biomass according to the process defined in any of claims 5 to 14;
(b) Pressing
(c) Addition of the additional components
(d) Heating and
(e) Freezing.

19. PRODUCT PRODUCTION PROCESS, according to claim 18, **CHARACTERIZED BY** the step of adding additional components, the pressed biomass is submerged in a solution containing the flavoring, chickpea flour and gum arabic until the product absorbs 10% of its weight from the solution.
